Europäisches Patentamt

European Patent Office

Office européen des brevets

(19)

(11) Publication number: **0 243 496**
A1

## (12) EUROPEAN PATENT APPLICATION
published in accordance with Art. 158(3) EPC

(21) Application number: 85904153.5

(22) Date of filing: 26.08.85

Data of the international application taken as a basis:

(86) International application number:
PCT/JP85/00468

(87) International publication number:
WO87/01390 (12.03.87 87/06)

(51) Int. Cl.³: **C 12 P 13/08**
C 12 N 1/20
//(C12P13/08, C12R1:01,
C12N1:20, C12R1:01)

(43) Date of publication of application:
04.11.87 Bulletin 87/45

(84) Designated Contracting States:
DE FR GB IT

(71) Applicant: TORAY INDUSTRIES, INC.
2, Nihonbashi-Muromachi 2-chome Chuo-ku
Tokyo 103(JP)

(72) Inventor: YAMADA, Katsushige
115-6, Aza-Kita Oaza-Nakanogo Nishiharucho
Nishikasugai-gun Aichi 481(JP)

(72) Inventor: YOTSUMOTO, Kyousuke
Room 2, Toray Wakakusa Shataku 5, Wakakusacho
Minami-ku Nagoya-shi Aichi 457(JP)

(74) Representative: Kador & Partner
Corneliusstrasse 15
D-8000 München 5(DE)

(54) PROCESS FOR PRODUCING L-THREONINE BY FERMENTATION.

(57) L-Threonine can be accumulated in a remarkable amount by culturing a microorganism belonging to the genus *Providensia* and having resistance to methionine antimetabolite and L-threonine-yielding ability.

EP 0 243 496 A1

Croydon Printing Company Ltd.

SPECIFICATION

-

## PROCESS FOR PRODUCING L-THREONINE BY FERMENTATION

### FIELD OF ART

This invention relates to a process for producing L-threonine by fermentation.

### BACKGROUND ART

Hitherto, the methods using L-isoleucine requiring mutant (Japanese Examined Patent Publication No. 4440/1968) or using the mutant having a resistance to $\alpha$-amino-$\beta$-hydroxyvaleric acid and requiring L-isoleucine ((Abs.) Ann. Meet. Agric. Chem. Soc. Japan, p 9, (1970)) have been known with respect to production of L-threonine by fermentation with using certain microorganisms of the genus Providencia: the species rettgeri was classified as genus Proteus before.

However, there is a room for further improvement in the capability of the strains as to the accumulation of L-threonine in the method using the above-mentioned microorganisms.

0243496

## DISCLOSURE OF THE INVENTION

As a rusult of the present inventors' earnest investigation for more profitable production of L-threonine by fermentation process, the inventors have found that certain mutants of genus Providencia which are resistant to methionine antagonist, accumulate a large amount of L-threonine. Thereby the inventors have accomplished the present invention.

Namely, the present invention is a process for producing L-threonine by fermentation comprising

(a) cultivating L-threonine-producing microorganism belonging to the genus Providencia until L-threonine is accumulated in a culture medium, said microorganism having a resistance to methionine antagonist and

(b) recovering the L-threonine from the culture broth.

The methionine antagonist-resistant mutant of the genus Providencia has not been yet isolated. Moreover, there has not been known at all that a large amount of L-threonine can be secreted and accumulated in the culture broth by the methionine antagonist-resistant mutant belonging to the genus Providencia.

## THE BEST FORMS TO PRACTICE THE INVENTION

The present invention will be described in further details hereinafter.

The microorganisms used in the invention belong to the genus Providencia (the genus is decided according to Bergy's Manual of Systematic Bacteriology, the 9th edition, pages 494 to 496) and have a resistance to methionine antagonist. In the invention, preferable examples of the methionine antagonists are ethionine, norleucine, crotonylalanine, crotonylglycine, methionine sulfoxime, and so on. The microoragisms used in the present invention include the strains which have at least the above-mentioned character, even though these stains have other requirements for growth thereof or a resistance to other chemical compounds.

The preferable microorganisms employed in the invention require L-isoleucine and have a resistance to feedback control by L-threonine in addition to the above-mentioned resistance.

That is, in the invention there can be used methionine antagonist-resistant mutants which acquire the effective charactors for L-threonine secretion, for examples, requiring L-isoleucine,

having a resistance to α-amino-β-hydroxyvaleric acid through the conventional artificial mutation.

In the present invention, requiring L-isoleucine for the growth thereof means wide concept and includes leaky type, namely incomplete defect type, and further includes the case when growth requirement is satisfied with biosynthetic precursors of L-isoleucine.

The specimens of the mioroorganisms used in the present invention are as follows:

(a) Providencia rettgeri TY-1 (FERM BP-871)

(b) Providencia rettgeri TY-2 (FERM BP-872).

These mutants have a resistance to L-ethionine among methione analogs and were derived from Providencia rettgeri IN4-7H (having a resistance to α-amino-β-hydroxyvaleric acid, requiring L-isoleucine). Providencia rettgeri IN4-7H was derived from Providencia rettgeri ATCC 21118 (requiring L-isoleucine).

These mutants can be relatively easily obtained by conventional mutation methods. Namely, in the case of obtaining the methionine antagonist-resistant mutant, the parental cells is irradiated with ultraviolet light or treated with mutagene (for example, N-methyl-N'-nitro-N-nitrosoguanidine or ethylmethane sulfonate), and said resistant mutant is obtained as good grower on the agar plate containing so high concentration of methionine antagonist to prevent the parental strain from growing.

The strain having a resistance to methionine antagonists in the present invention, is defined as a strain of which growth degree in the culture medium supplemented with 4 mg/ml of methionine antagonist is at least 50 %, preferably at least 70 %, based on the case in the absence of methionine antagonist. In the invention, growth degree is shown by the relative optical density of culture broth at 660 nm when the optical density of culture broth in none-supplement of methionine antagonist is defined as 100 %. Methionine antagonists such as ethionine to examine resistance can be commercially available materials.

In the present invention, L-threonine producing culture medium is conventional medium containing carbon source, nitrogen source, inorganic ions, and if necessary, other organic minor ingredients.

The preferable culture medium may contain 2 to 15 % of carbon sources, for examples, carbohydrates such as glucose, fructose, hydrolysate of starch or cellulose, or molasses; organic acids such as fumalic acid, citric acid, or succinic acid; alcohols such as glyceral and contain 0.5 to 4.0 % of nitrogen source, for examples, organic ammonium salts such as ammonium acetate; inorganic ammonium salts such as ammonium sulfate, ammonium chloride, ammonium phosphate, or ammonium nitrate; ammonia gas; aqueous ammonia; urea, and contain 0.001 to 0.4 % of required materials such as L-isoleucine, and if necessary, contain 0 to 4 % of corn steep liquor, polypeptone, or yeast extract and the like as an organic minor nutrient. In addition, small amount of potassium phosphate, magnesium sulfate, ferrous sulfate 7-hydrate, 4- to 6-hydrate of manganese sulfate, etc. may be added to culture medium.

Cultivation is carried out preferably under aerobic conditions. Preferable result is obtained by adjusting the pH of the medium to from 5 to 9, and controlling temperature from 24 to 37 ℃ during cultivation, and shaking or stirring with aeration for 48 to 120 hours.

0243496

An optional process, for cultivation which includes, for example, any of continuous operation, semi-continuous operation and batch operation, can be employed.

The recovery of L-threonine from culture broth is carried out, for example, by following method.

The culture broth from which the cells are removed is adjusted to pH 2 with hydrochloric acid, then the broth solution is passed through the strongly acidic ion exchange resin, and the adsorbant is eluted by dilute aqueous ammonia. Ammonia is evaporated and then the resulting solution is condensed. Alcohol is added to the concentrate, and then, crystals formed under cooling are collected, and then L-threonine can be obtained.

Example 1

A. (Isolation of the mutant strains having a resistance to L-ethionine.)

The cells of Providencia rettgeri IN4-7H(which has a resistance to $\alpha$-amino-$\beta$-hydroxyvaleric acid and requires L-isoleucine for growth) were irradiated with ultraviolet light by a conventional method, and these cells were spread on the agar plate (which contained glucose 1.0 %, ammonium sulfate 0.3 %, patassium monohydrogen phosphate 0.05 %, potassium dihydrogen phosphate 0.15 %, magnesium sulfate 7-hydrate 0.04 %, L-isoleucine 0.01 %) supplied with 10 g/ℓ L-ethionine. Then, after incubation for 4 to 6 days at 30 ℃ large colonies formed on the plate were picked up, and L-ethionine-resistant strains (Providencia rettgeri TY-1 and TY-2) were obtained.

B. (Degree of resistance of L-ethionine-resistant mutants)

Each strain shown in Table 1 was cultivated in bouillon liquid medium at 30 ℃ for 16 hours with shaking, and the grown cells were harvested and washed with physiological saline.

The resulting cell suspension was inoculated into 5 mℓ of the minimal medium (composition: glcose 1.0 %, ammonium sulfate 0.3 %, potassium monohydrogen phosphate 0.05 %, potassium dihydrogen phosphate 0.15 %, magnesium sulfate 7-hydrate 0.01 %, L-isoleucine 0.01 %) containing 0 g/ℓ, 4 g/ℓ, 8 g/ℓ, 16 g/ℓ, of L-ethionine, respectively, and cultivated at 30 ℃ for 24 hours, and the growth degree of each strain was measured.

The results are shown in Table 1, and the growth of L-ethionine-resistant mutants (Providencia rettgeri TY-1 and TY-2) used in the present invention are not inhibited in the presence

0243496

of the high concentration of L-ethionine.  These
mutants, therefore, have a strong resistance to
L-ethionine.

Table 1

| Strains | Relative growth degree[*] (%) | | | |
| --- | --- | --- | --- | --- |
| | Amount of L-ethionine added (g/ℓ) | | | |
| | 0 | 4 | 8 | 16 |
| Parent strain | | | | |
| Providencia rettgeri IN4-7H | 100 | 18 | 2 | 0 |
| This invention strains | | | | |
| Providencia rettgeri TY-1 | 100 | 79 | 77 | 71 |
| Providencia rettgeri TY-2 | 100 | 84 | 97 | 80 |

[*) Relative growth degree is shown by the relative
optical density of the culture broth at 660 nm
when the optical density of the culture broth
in the absence of L-ethionine is 100 %.

- 9 -

Example 2

The 50 ㎖ of fermentation medium, which has the following composition, in 1-liter erlenmeyer flask was sterilized at 120 ℃ for 10 minutes.

The 5 ㎖ of culture broth of each strain shown in Table 2, which was cultivated at 30 ℃ for 16 hours with shaking in a seed culture medium containing 2 % of glucose, 1 % of polypeptone, 1 % of yeast extract and 0. 5 % of NaCl, was put into the fermentation medium and then was cultivated at 30 ℃ for 90 hours with shaking condition of 150 rpm and 3 ㎝-stroke.

<u>Fermentation medium composition</u>

| | |
|---|---|
| Glucose | 8 % |
| $(NH_4)_2SO_2$ | 2 % |
| $KH_2PO_4$ | 0. 1 % |
| $MgSO_4 \cdot 7H_2O$ | 0. 04 % |
| $Fe^{++}$ | 2 ppm |
| $Mn^{++}$ | 2 ppm |
| L-isoleucine | 0. 005 % |
| $CaCO_3$ | 3 % |
| (sterilized separately) | |
| pH | 7. 0 |

(neutralized with KOH)

Table 2

| | Strain | Amount of L-threonine accumulated $(g/\ell)$ |
|---|---|---|
| Comparative Example | Providencia rettgeri IN4-7H | 6.0 |
| This invention Examples | Providencia rettgeri TY-1 | 10.6 |
| | Providencia rettgeri TY-2 | 10.4 |

After cultivation, the amount of L-threonine in the filtrate which was obtained by removing the cells and calcium carbonate from culture broth was quantitatively analyzed by automatic amino acid analyzer (produced by Japan Electric Co. JLC-200A), and the results shown in Table 2 were obtained.

Example 3

Each strain shown in Table 3 was cultivated in bouillon liquid medium at 30 ℃ for 16 hours with shaking, and this culture broth was inoculated by 10 % by volume into a small glass jar fermentor containing 800 ㎖ of the same fermentation medium as used in Example 2 except that 0.5 % of

$(NH_4)_2SO_4$ and 4.0 % of glucose were used. Cultivation with aeration (1 vvm) and agitation (800 rpm) was started at 30 °C.

Controll of pH and feed of nitrogen source were served with 25 % aqueous ammonia and pH was kept from 6.5 to 8.0. Cultivation was carried out with intermittent feeding of glucose, $KH_2PO_4$ and $MgSO_4 \cdot 7H_2O$ for 70 hours and the results shown in Table 3 were obtained.

Table 3

|  | Strain | Amount of L-threonine accumulated $(g/ℓ)$ |
|---|---|---|
| Comparative Example | Providencia rettgeri IN4-7H | 8.1 |
| This invention Example | Providencia rettgeri TY-1 | 19.0 |

The cells were removed from the culture broth of Providencia rettgeri TY-1. 500 mℓ of the resulting filtrate was passed through the column packed with strong cation exchange resin DIAION (Trade Name) SK·1B (H type). Then, the column was washed with water and thereafter the adsorbant in the column was eluted by 2 N aqueous ammonia.
The eluent was concentrated under reduced pressure
after decoloriging.  Ethanol was added to the
resultant and left standing under cooling, and
then the crystals formed were collected, dried to
give 8.4 g of L-threonine having over 96 % of
purity.

INDUSTRIAL APPLICABILITY OF THE INVENTION

L-threonine is one of the essential amino
acids and known to be important as medicine and
feed additive.

CLAIMS

(1) A process for producing L-threonine by fermentation comprising

(a) cultivating L-threonine-producing microorganism belonging to the genus Providencia until L-threonine is accumulated in a culture medium, said microorganism having a resistance to methionine antagonist and

(b) recovering the L-threonine from the culture broth.

(2) A microorganism belonging to the genus Providencia, and having a resistance to methionine antagonist, and having capabilities of producing L-threonine.

(3) A microorganism according to Claim 2, wherein said microorganism is characterized in having a resistance to α-amino-β-hydroxyvaleric acid and requiring L-isoleucine for growth.

0243496

# INTERNATIONAL SEARCH REPORT

International Application No. PCT/JP85/00468

## I. CLASSIFICATION OF SUBJECT MATTER (if several classification symbols apply, indicate all) [3]

According to International Patent Classification (IPC) or to both National Classification and IPC

Int. Cl[4] C12P 13/08, C12N 1/20 // (C12P 13/08, C12R 1:01) (C12N 1/20, C12R 1:01)

## II. FIELDS SEARCHED

### Minimum Documentation Searched [4]

| Classification System | Classification Symbols |
|---|---|
| IPC | C12P 13/08, C12N 1/20 |

Documentation Searched other than Minimum Documentation
to the Extent that such Documents are included in the Fields Searched [5]

## III. DOCUMENTS CONSIDERED TO BE RELEVANT [14]

| Category* | Citation of Document, [16] with indication, where appropriate, of the relevant passages [17] | Relevant to Claim No. [18] |
|---|---|---|
| A | JP, B2, 47-10999 (Kyowa Hakko Kogyo Co., Ltd.) 3. April. 1972 (03.04.72) (Family: none) | 1 |
| A | JP, B1, 45-26709 (Ajinomoto Co., Inc.) 2. September, 1970 (02.09.70) (Family: none) | 1 |
| A | JP, B1, 43-4440 (Chugai Pharmaceutical Co., Ltd.) 17. February. 1968 (17.02.68) (Family: none) | 1 |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search [2] | Date of Mailing of this International Search Report [2] |
|---|---|
| November 18, 1985 (18.11.85) | November 25, 1985 (25.11.85) |

| International Searching Authority [1] | Signature of Authorized Officer [20] |
|---|---|
| Japanese Patent Office | |